# EUROPEAN PATENT APPLICATION

(11) **EP 2 286 835 A1**
(43) Date of publication of application: **23.02.2011**
(21) Application number: 10180347.6
(22) Date of filing: 30.09.2005
(51) Int. Cl.: A61K 39/395

(54) **Type I interferon blocking agents for prevention and treatment of psoriasis**

(30) Priority: 07.10.2004 EP 04405628
(62) Divisional of application: 05786466.2
(71) Applicant: Universität Zürich Prorektorat Forschung, 8006 Zürich (CH)
(72) Inventor: Gilliet, Michel, Pearland, 77584 (US); Nestle, Frank, O, NewYork, 10021 (US)
(74) Representative: Sampson, Catherine

(57) **Abstract**

The discovery of plasmacytoid dendritic cell precurosors (PDC) as crucial effector cells with high production of type I interferon (IFNs) in early psoriasis development has led to the present invention that blocking of type I IFNs can be used for prevention and therapy of psoriasis. The invention relates to the use of a type I interferon clocking agent, such as a type I IFN antagonist (e.g. an anti-IFN-α antibody) or type I IFN receptor antagonist, for the preparation of a medicament for the prevention and treatment of psoriasis, and to a method of prevention and treatment of psoriasis using a type I interferon blocking agent.

## Description

### Field of the invention

The invention relates to the use of a type I interferon blocking agent for the preparation of a medicament for the prevention and treatment of psoriasis, and to a method of prevention and treatment of psoriasis using a type I interferon blocking agent.

### Background of the invention

Psoriasis is a common autoimmune-related inflammatory disease affecting the human skin. There is compelling evidence that, similarly to Crohn's disease and rheumatoid arthritis, psoriasis formation results from an overt self-perpetuating activation of autoreactive IFN-γ secreting T cells. The initial onset of skin lesions is followed by chronic relapses of the disease, typically triggered by environmental factors including infections, mechanical stress and drugs. It has been proposed that these insults may drive the pathogenic T cell cascade through a yet unidentified innate immune response.

Plasmacytoid dendritic cell precursors (PDC) are key effectors in innate antiviral immunity due to their unique ability to secrete large amounts of type I interferons (IFN-α/β) in response to viral stimulation. Virally exposed PDC subsequently differentiate into T cell stimulatory dendritic cells (DC) themselves or induce maturation of bystander myeloid DC through IFN-α, thus providing a unique link between innate and adaptive anti-viral immunity. During homeostasis, PDC are encountered exclusively in blood and lymphoid organs, however viral infection leads to active recruitment of PDC from the blood into peripheral sites of primary infection. Wollenberg et al. [J Invest Dermatol 2002, 119: 1096-102] have shown that PDC may also accumulate in peripheral tissues of certain non-infectious inflammatory disorders such as allergic contact dermatitis, cutaneous lupus erythematosus and psoriasis, however a functional relevance for PDC or their secreted products such as type I IFNs has not been addressed or proven so far.

Type I (IFN-α, IFN-β, IFN-ω) IFNs are members of a cytokine family including several structurally related IFN-α proteins and a single IFN-β protein binding to the type I IFN surface receptor. Type I IFNs inhibit viral replication, increase the lytic potential of NK cells, increase expression of class I MHC molecules and stimulate the development of T helper 1 cells in humans.

In the past, there have been some reports in the literature dealing with the association between psoriasis and type I IFNs or the description of IFN-α blocking agents. Schmid et al. [J of IFN Res 1994, 14: 229-234] have detected low levels of IFN-α mRNA expression in psoriasis epidermis. However, they failed to show IFN-α expression on the protein level. Furthermore IFN-α was found only in the epidermal compartment. Although the authors provided direct evidence that the IFN-α system is locally activated in psoriasis they concluded that this might be the result of a viral infection or a dysregulation of the cytokine network.

Van der Fits et al. [J Invest Dermatol 2004, 122: 51-60] describe an activated type I interferon signalling pathway in psoriatic lesional skin, but do not suggest that blocking of this pathway could be used for prevention or therapy of psoriasis. Single case reports have indicated that systemic IFN-α given during adjuvant therapy of melanoma or hepatitis therapy can occasionally trigger psoriasis in predisposed individuals [Pauluzzi et al., Acta Derm Venereol 1993, 73: 395; Funk et al., Br J Dermatol 1991, 125: 463-5]. However, this is a rare event considering the high prevalence of psoriasis and the frequent use of IFN-α in cancer patients as well as in anti-infectious therapy. Furthermore, IFN-α is just thought of being one of many factors able to induce psoriasis. Other factors such as physical and psychological stress, HIV infection as well as various medications including lithium, beta blockers and anti-malarial drugs are also well-known triggers of psoriasis. Therapeutic IFN-α doses, added through the exogenous route, might be a nonspecific trigger of several possible downstream psoriasis inducing factors.

Chuntharapai et al. [Cytokine 2001, 15: 250-260] focus on the development of a therapeutic agent that neutralizes IFN-α, i.e. the development of a humanized antibody. No experimental data are provided to show prevention or therapy in any of the known autoimmune diseases, although insulin-dependent diabetes mellitus (IDDM) or systemic lupus erythematosis (SLE) are mentioned as potential diseases to be treated. The paper questions the statement about an association between IFN-α and psoriasis or Crohn's disease by referring to the limited number of patients having been analyzed.

WO 00/64936 [Wieser] describes peptide homodimers and peptide heterodimers binding to the IFN-α 2 receptor and focuses on the physical and biochemical activities of these compounds representing IFN-α 2 substitutes. Application of these compounds to inflammatory and neoplastic diseases in the broadest sense are suggested, and the list of diseases also contains psoriasis. However, there is no indication that IFN-α 2 antagonists should be used.

There is an unmet need for novel psoriasis therapies since current therapies of psoriasis are limited by their limited efficacy, their side effects and their inability to prevent new relapses. While novel drugs such as anti-TNF-α targeted therapies are able to induce fast disease remission, long term therapy is not an option due to their high potential toxicity.

### Summary of the invention

The invention relates to the use of a type I interferon blocking agent, such as a type I IFN antagonist or type I IFN-receptor antagonist, for the preparation of a medicament for the prevention and treatment of psoriasis, and to a method of prevention and treatment of psoriasis using a type I interferon blocking agent.

In particular, the invention relates to such a use, wherein the type I IFN antagonist is an IFN-α antagonist, for example an anti-IFN-α antibody or antibody fragment, preferably a humanized antibody, a type I IFN receptor fusion protein, or also short interfering (si) RNA or antisense oligonucleotides inhibiting IFN-α production by sequence-specific targeting of IFN-α mRNA. Similar reagents antagonizing other type I IFN family members or groups of type I IFNs are also part of the invention. The invention further relates to such a use, wherein the type I IFN-receptor antagonist is an anti-IFN-α/β-recepto antibody or antibody fragment, mutant type I IFN/Fc fusion protein or small molecule specifically interfering with type I IFN signalling.

### Brief description of the figures

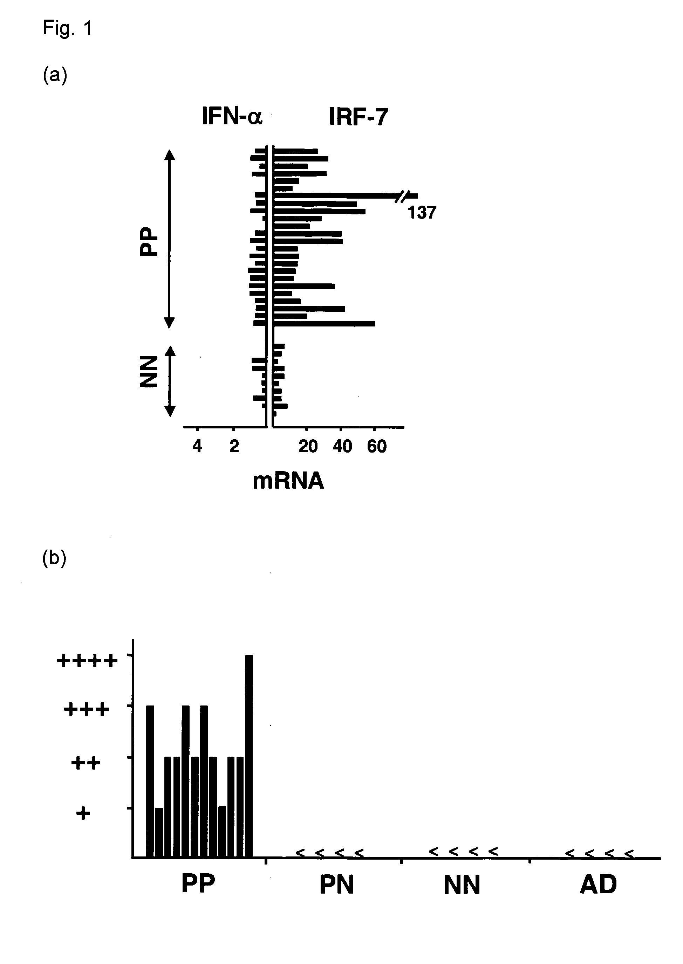

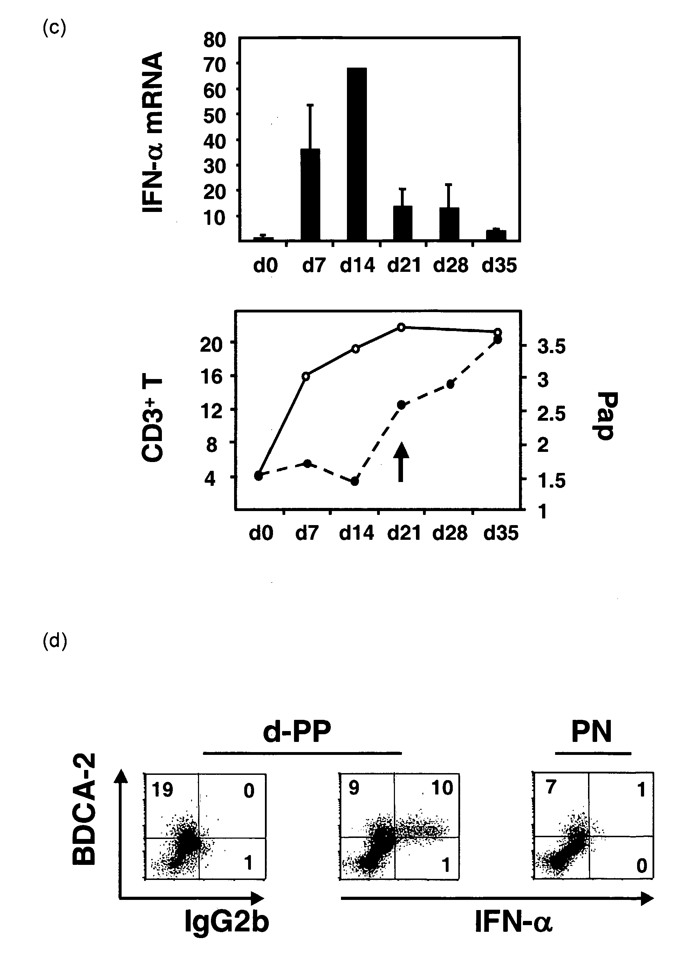

Figure 1. *IFN-*α *production is an early event during the development of psoriatic skin lesions and is principally mediated by dermal PDC.*
(a) Normalized IFN-α and IRF-7 mRNA expression in psoriatic plaque lesions (PP, n=24) and normal skin (NN, n=11). The figures of the x axis (mRNA) represent relative mRNA expression.
(b) Immunohistochemistry for MxA protein on cryosections of psoriatic plaque lesions (PP, n=8), uninvolved skin of psoriatic patients (PN, n=4), normal skin of healthy individuals (NN, n=4) and atopic dermatitis skin (AD, n=4) specimen. Percentages of MxA-positive cells among the total dermal and epidermal cells are calculated from mean of two independent counts of three random fields with a 400-fold magnification. + = 0-25%, ++ = 25-50%, +++ = 50-75%, ++++ = 75-100%, < = below detection limit.
(c) Lesional IFN-α expression during the spontaneous development of a psoriatic lesion from uninvolved skin transplanted onto AGR^{-/-} mice. Kinetics of human IFN-α mRNA expression relative to human GAPDH mRNA (IFN-α mRNA, bars) in comparison to the expansion of resident CD3 T cells (CD3, solid line) and the induction of psoriatic papillomatosis (Pap, dashed line). d=days after transplantation. Data represent the mean +/- standard deviation (SD) of two independent experiments.
(d) Double staining of intracellular IFN-α and surface BDCA-2 in dermal single cell suspensions derived from developing psoriatic lesions (d-PP, advancing edges of a psoriatic plaques) and uninvolved skin (PN). Figures in the quadrants represent %.

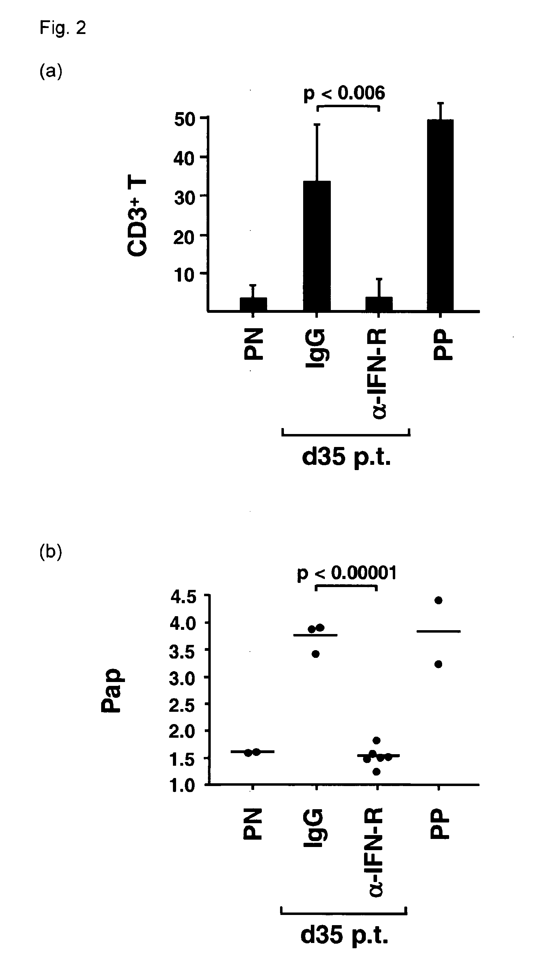

Figure 2. *IFN-*α/β *signalling is crucial for local T cell expansion and the development of psoriasis.*
(a) Total CD3 T cell count and (b) epidermal papillomatosis (Pap) in skin grafts before transplantation (uninvolved skin day 0, PN) and 35 days post transplantation (p.t.) following the administration of isotype-matched control antibody (IgG) or an anti-IFN-α/β-receptor mAb (α-IFN-R), or in a psoriatic plaque (PP) of the graft donor. Error bars in (a) represent one standard deviation (SD). Dots in (b) represent independently grafted mice.

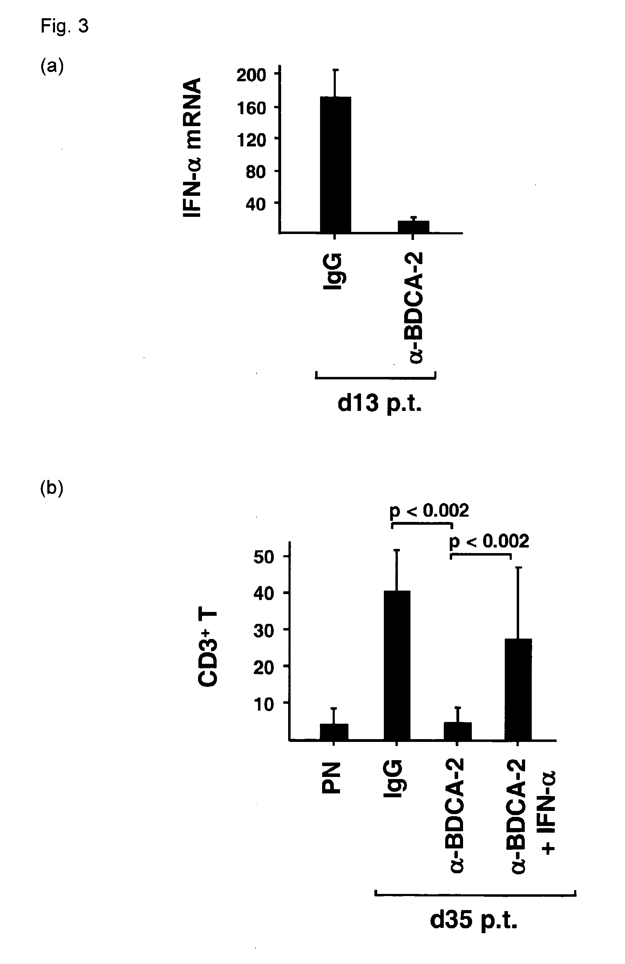

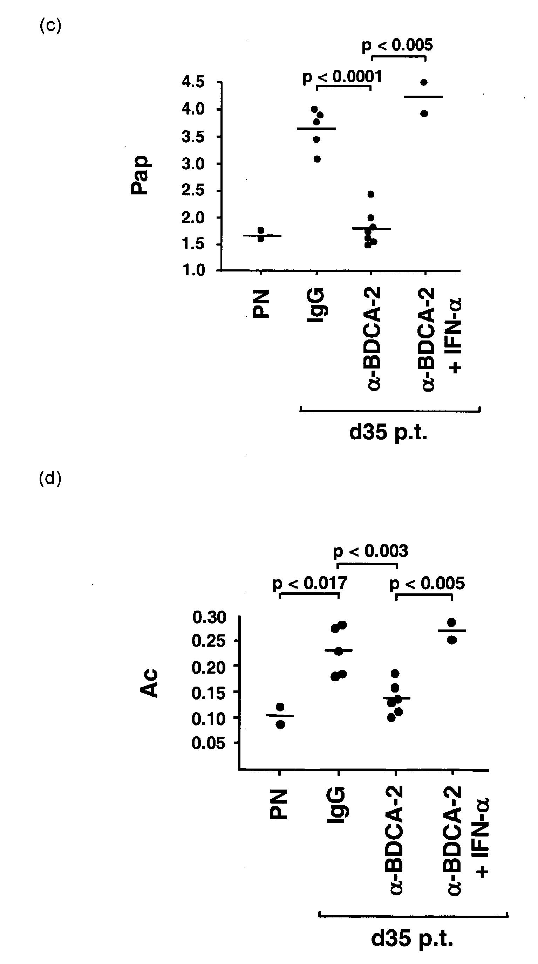

Figure 3. *The development of psoriasis is dependent on type I IFN production by PDC.*
(a) Total human IFN-α mRNA expression relative to human GAPDH mRNA in skin grafts harvested 13 days post transplantation (p.t.) following the administration of isotype-control Ab (IgG) or anti-BDCA-2 mAb (α-BDCA-2).
(b) Total CD3 T cell count, (c) epidermal papillomatosis (Pap) and (d) acanthosis (Ac) in skin grafts before transplantation (uninvolved skin d 0, PN) and 35 days post transplantation (p.t.) following the administration of isotype-matched control Ab (IgG), anti-BDCA-2 mAb (α-BDCA-2), or anti-BDCA2 plus human recombinant IFN-α2 (α-BDCA-2 + IFN-α).

### Detailed description of the invention

The discovery of PDC as crucial effector cells with high production of type I IFNs in early psoriasis development has led to the present invention that blocking of type I IFNs can be used as prevention and therapy of psoriasis. High numbers of PDC infiltrating the skin of both psoriatic plaque lesions as well as uninvolved (normal appearing) skin of psoriatic patients can be shown by immunohistochemistry and flow cytometry of single cell suspensions using an antibody specific for PDC (anti-BDCA-2). Interestingly, in contrast to the resting phenotype of PDC in uninvolved skin, PDC infiltrating psoriatic plaque lesions display an activated phenotype. PDC activation during the transition from uninvolved skin into psoriatic plaque lesions contributes to the pathogenesis of psoriasis.

Antagonizing type I IFNs (e.g. antagonizing IFN-α, while leaving IFN-β intact) provides the unique opportunity to specifically target the type I IFN mediated autoimmune disease process in psoriasis while potentially leaving an important IFN-β mediated antiviral immune response intact. Antagonizing IFN-α not only clears the disease, but also prevents relapse as is demonstrated in a relevant preclinical model. The AGR psoriasis mouse model [Boyman et al., J Exp Med 2004, 199: 731-736] provides for the first time the platform to prove the effectiveness of type I IFN blocking in prevention and therapy of psoriasis. This clinically relevant psoriasis model supports that inhibiting type I IFN blocks development of psoriasis and is a potent way to prevent and treat psoriasis in humans.

IFN-α expression during the development of psoriatic lesions is studied in a xenotransplantation model, in which uninvolved skin of psoriatic patients transplanted onto Agar^{-/-} mice spontaneously converts into a fully-fledged psoriatic skin lesion within 35 days. AGR129 mice, deficient in type I (A) and type II (G) IFN receptor in addition to being RAG-/-, are kept pathogen-free throughout the study. Keratomes of uninvolved skin are transplanted to the back of mice using an absorbable tissue seal. This humanized mouse model system is dependent on the local activation and proliferation of resident human T cells derived from the engrafted pre-psoriatic skin.

Initial screening of psoriatic plaque lesions does not show significant upregulation of IFN-α mRNA compared to normal skin of healthy donors. However, psoriatic plaque lesions but not uninvolved skin or normal skin demonstrate an IFN-α signature with significantly increased expression of IRF-7, an IFN-α inducible gene (Fig. 1a), and the presence of the IFN-α inducible MxA protein (Fig. 1b), suggesting that IFN-α is produced earlier during the development of the psoriatic phenotype. Analysis of human IFN-α expression reveals increased mRNA levels as early as day 7 after engraftment, reaching a peak at day 14, before rapidly declining (Fig. 1c). The induction of IFN-α expression at day 7 and 14 is paralleled by the local expansion of resident T cells. In contrast, disease formation, quantified by epidermal papillomatosis, shows delayed kinetics, starting at day 21 after transplantation and reaching its full development at day 35 (Fig. 1c). These data indicate that IFN-α expression is especially important during the early phase of the development of the psoriatic phenotype but has consequences for the whole disease process.

Intracellular IFN-α expression is confined to BDCA2+ cells (Fig. 1d), indicating that PDC represent the principal IFN-α producers in developing psoriatic skin lesions. By contrast, IFN-α expression is not detectable on PDC derived from uninvolved skin nor in peripheral blood of the same psoriasis patient. Since PDCs contain high amounts of type I interferons, PDC-derived IFN-α plays a crucial role in the elicitation of psoriasis.

Intravenous injection of neutralizing anti-IFN-α/β-receptor antibody, starting immediately after transplantation, inhibits the local activation and expansion of resident T cells (Fig. 2a), and completely blocks the development of the psoriatic phenotype, with a significant reduced papillomatosis (Fig. 2b) compared to mice receiving the isotype-matched control antibody.

The spontaneous conversion of uninvolved pre-psoriatic skin to psoriasis in AGR^{-/-} mice is mediated by PDC activation and the secretion of IFN-α. Anti-BDCA2 antibody specifically targets human PDC and inhibits type I IFN-production by PDC *in vitro.* Intravenous injection of anti-BDCA2 monoclonal antibody (mAb) leads to a 14-fold reduction of lesional IFN-α at day 13 after transplantation (Fig. 3a), inhibits the dermal T cell expansion (Fig. 3b) and the development of the psoriasis phenotype, quantified by epidermal papillomatosis (Fig. 3c) and acanthosis (Fig. 3d).

Addition of exogenous IFN-α to the PDC-blocking by anti-BDCA2 treatment completely reverses the inhibition of T cell expansion (Fig. 3b) and induction of psoriasis development (Fig. 3c, d), confirming that the development of psoriasis is mediated by IFN-α production by PDC. These data prove that IFN-α is the responsible type IFN for psoriasis development.

Specific blockade of IFN-α blocks psoriasis while leaving IFN-β signalling intact for a potential antiviral immune response.

Blocking agents refer to any DNA, RNA (si RNA, antisense molecules), peptide, protein (including fusion protein), antibody or small molecules interfering with type I IFN signalling and function and/or type I IFN production by PDCs in inflammatory diseases. In particular such blocking agents are (i) humanized or human anti-IFN-α whole antibodies or antibody fragments (Fab or scFv), antagonizing secreted IFN-α, (ii) short interfering (si) RNA or antisense oligonucleotides inhibiting IFN-α production, (iii) anti-IFN-α/β-receptor antibodies, mutant type I IFN/Fc fusion proteins, type I IFN receptor fusion proteins, or small molecules interfering with type I IFN signalling.

One aspect of the invention relates to a method of treating psoriasis comprising administering an anti-IFN-α antibody or antibody fragment in a quantity effective against psoriasis to a mammal in need thereof, for example to a human requiring such treatment. The treatment may be for prophylactic or therapeutic purposes. For the administration, the anti-IFN-α antibody is preferably in the form of a pharmaceutical preparation comprising the anti-IFN-α antibody and optionally a pharmaceutically acceptable carrier and optionally adjuvants. The anti-IFN-α antibody is used in an amount effective against psoriasis. The dosage of the active ingredient depends upon the species, its age, weight, and individual condition, the individual pharmacokinetic data, the mode of administration, and whether the administration is for prophylactic or therapeutic purposes. In the case of an individual having a bodyweight of about 70 kg the daily dose administered is from approximately 1 mg to approximately 500 mg, preferably from approximately 10 mg to approximately 100 mg, of an anti-IFN-α antibody.

Administering other blocking agents referred to above is also included in the invention, in particular administering pharmaceutical preparations comprising short interfering (si) RNA or antisense oligonucleotides inhibiting IFN-α production, or anti-IFN-α/β-receptor antibodies, mutant type I IFN/Fc fusion proteins, type I IFN receptor fusion proteins or small molecules interfering with type I IFN signalling. These compounds are likewise used in an amount effective against psoriasis. The dosage is chosen by the practitioner based on the particular compound to be administrated and the individual pharmacokinetic data, and also on the species, its age, weight, and individual condition and the mode of administration.

Pharmaceutical compositions for parenteral administration, such as intravenous, intramuscular or subcutaneous administration, are especially preferred. The pharmaceutical compositions comprise from approximately 1% to approximately 95% active ingredient, preferably from approximately 20% to approximately 90% active ingredient.

For parenteral administration preference is given to the use of suspensions or dispersions of the anti-IFN-α antibody or other type I IFN blocking agent mentioned above, especially in isotonic aqueous solutions, which, for example, can be made up shortly before use. The pharmaceutical compositions may be sterilized and/or may comprise excipients, for example preservatives, stabilizers, wetting agents and/or emulsifiers, solubilizers, viscosity-increasing agents, salts for regulating osmotic pressure and/or buffers and are prepared in a manner known *per se,* for example by means of conventional dissolving and lyophilizing processes.

An antibody useful in the invention is prepared by standard methods. Humanized antibodies and antibody fragments are obtained by recombinant technologies as previously described [reviewed by Dall'Acqua et al., Curr Op Struct Biol 1988, 8: 443-50]. Alternatively, entirely human antibodies can be obtained using either phage display technologies [reviewed by Winter et al., Annu Rev Immunol 1994, 12: 433-55] or transgenic "human" mice with partial human heavy and light chain loci inserted into their genomes [reviewed by Brüggeman et al., Curr Op Biotechnol 1997, 8: 503-8]. Preparation of si RNA or antisense oligonucleotides likewise uses standard technology.

An anti-IFN-α antibody or other type I IFN blocking agent mentioned above can be administered alone or in combination with one or more other therapeutic agents, possible combination therapy taking the form of fixed combinations of a blocking agent of the invention and one or more other therapeutic agents known in the treatment of psoriasis, the administration being staggered or given independently of one another, or being in the form of a fixed combination.

Combination partners considered are topical corticosteroids, UV light, retinoids, methotrexate, other biologics targeting the altered immune system in psoriasis or derivatives of vitamin D3.

The following Examples serve to illustrate the invention without limiting the invention in its scope.

### Examples

*Real time quantitative PCR.* Total RNA from homogenized skin specimens is extracted and reverse transcribed as previously described [Boyman et al., J Exp Med 2004, 199: 731-736]. Complementary DNA is quantitatively analyzed for the expression of IFN-α and IRF-7 transcripts by real time PCR, using primers designed against most human IFN-α sequences (purchased from Applied Biosystems, Foster City, CA) and against human IRF-7 (left, TCCCCACGCTATACCATCTACCT-3'; right, ACAGCCAGGGTTCCAGCTT-3'). 18S ribosomal RNA is used for normalisation. In the humanized mouse model IFN-α quantification is done by using a primer kit recognizing most human IFN-α genes and which does not recognize its mouse counterpart (purchased from Search-LC, Heidelberg, Germany). Human GAPDH mRNA levels are quantified using human-specific primers (left, ATTGCCCTCAACGACCACTTTG-3'; right, TTGATGGTACATGAAAGGTGAGG-3') and used for normalization.

*Animals, and Transplantation Procedure.* AGR129 mice, deficient in type I (A) and type II (G) IFN receptors in addition to being RAG-2^{-/-}, are kept pathogen free throughout the study. Keratomes of symptomless pre-psoriatic skin are transplanted to the back of mice using an absorbable tissue seal, as previously described [Boyman et al., *loc*. *cit.*]. 35 days after engraftment transplanted skin is removed and snap frozen for histological or mRNA expression analysis. CD3+ T cell counts, acanthosis and papillomatosis index are determined histologically as previously published [Boyman et al., *loc*. *cit*.]. CD3+ T cell values represent the mean cell count of three random fields assessed by a 400-fold magnification by two independent investigators. The indicated papillomatosis and acanthosis values represent the mean of 10 random areas of each sample.

*Neutralization Studies.* Dosage and schedule of antibody administration are deduced based on previous data with anti-human monoclonal antibodies against other cell surface molecules, and administered as follows: (i) intravenous injection of 30 µg neutralizing anti-human IFN-α/β Receptor Chain 2 (CD118) mAb (Clone MMHAR-2, purchased from PBL Biomedical Labortories) twice weekly for 35 days, starting at day 0 after transplantation; (ii) intravenous injection of 30 µg anti-BDCA-2 mAb (Miltenyi Biotech) twice weekly for 35 days, starting at day 0 after transplantation. For IFN-α reconstitution experiments, 30'000 IU recombinant human IFN-α2a (Roferon^{®}A, Roche Pharma AG, Reinach, Switzerland) are administered subcutaneously 3 times a week for 35 days. Dosage corresponds to the therapeutic dose of 8 Mio IU used in humans, and is deduced by an allometric approach as previously described [Boyman et al., *loc. cit*.].

Some additional embodiments of the invention are described in the following numbered paragraphs:
1. Use of a type I interferon blocking agent for the preparation of a medicament for the prevention and treatment of psoriasis.
2. Use according to paragraph 1 wherein the type I interferon blocking agent is a type I interferon antagonist.
3. Use according to paragraph 2 wherein the type I interferon antagonist is an IFN-α antagonist.
4. Use according to paragraph 3 wherein the IFN-α antagonist is an anti-IFN-α antibody or antibody fragment.
5. Use according to paragraph 1 wherein the type I interferon blocking agent is a type I interferon receptor fusion protein
6. Use according to paragraph 1 wherein the type I interferon blocking agent is a short interfering (si) RNA or an antisense oligonucleotide inhibiting IFN-α production.
7. Use according to paragraph 1 wherein the type I interferon blocking agent is a type I interferon receptor antagonist.
8. Use according to paragraph 7 wherein the type I interferon receptor antagonist is an anti-IFN-α/β-receptor antibody, mutant type I IFN/Fc fusion protein or small molecule specifically interfering with type I IFN signalling.
9. Use according to paragraph 7 wherein the type I interferon receptor antagonist is an anti- IFN-α/β-receptor antibody or antibody fragment.
10. Use according to paragraph 4 or 9 wherein the antibody is a humanized antibody.
11. A method of prevention and treatment of psoriasis using a type I interferon blocking agent.

## Claims

1. A type I interferon blocking agent for use in the prevention and treatment of psoriasis.

2. A type I interferon blocking agent according to claim 1 which is a type I interferon antagonist.

3. A type I interferon blocking agent according to claim 2 which is an IFN-α antagonist.

4. A type I interferon blocking agent according to claim 3 which is an anti-IFN-α antibody or antibody fragment.

5. A type I interferon blocking agent according to claim 1 which is a type I interferon receptor fusion protein.

6. A type I interferon blocking agent according to claim 1 which is a short interfering (si) RNA or an antisense oligonucleotide inhibiting IFN-α production.

7. A type I interferon blocking agent according to claim 1 which is a type I interferon receptor agonist.

8. A type I interferon blocking agent according to claim 7 which is an anti-IFN-α/β-receptor antibody, mutant type I IFN-Fc fusion protein or small molecule specifically interfering with type I IFN signalling.

9. A type I interferon blocking agent according to claim 7 which is an anti-IFN-α/β-receptor antibody or antibody fragment.

10. A type I interferon blocking agent according to claim 4 or 9 wherein the antibody is a humanized antibody.

11. Use of a type I interferon blocking agent for the preparation of a medicament for the prevention and treatment of psoriasis, wherein the type 1 interferon blocking agent is as defined in any one of claims.


SEQUENCE LISTING

<110>  Nestle, Frank O.
       Gilliet, Michel

<120>  Type I interferon blocking agents for prevention and treatment of
        psoriasis

<130>  UZ-06/167

<160>  4

<170>  PatentIn version 3.3

<210>  1
<211>  23
<212>  DNA
<213>  artificial

<220>
<223>  primer for IRF-7, left

<400>  1
tccccacgct ataccatcta cct                                              23


<210>  2
<211>  19
<212>  DNA
<213>  artificial

<220>
<223>  primer for IRF-7, right

<400>  2
acagccaggg ttccagctt                                                   19


<210>  3
<211>  22
<212>  DNA
<213>  artificial

<220>
<223>  primer for GAPDH, left

<400>  3
attgccctca acgaccactt tg                                               22


<210>  4
<211>  23
<212>  DNA
<213>  artificial

<220>
<223>  primer for GAPDH, right

<400>  4
ttgatggtac atgaaaggtg agg                                              23